# EUROPÄISCHE PATENTSCHRIFT

(11) **EP 0 723 686 B1**
(45) Veröffentlichungstag und Bekanntmachung des Hinweises auf die Patenterteilung: **23.04.1997**
(21) Anmeldenummer: 94928256.0
(22) Anmeldetag: 23.09.1994
(51) Int. Cl.: G06F 19/00, G06F 17/30

(54) **DIGITALES INTEGRATIONSSYSTEM ZUR INTEGRATION VON DIAGNOSTISCHEN BILDERZEUGUNGS- UND DATENVERARBEITUNGSGERÄTEN IN EDV-SYSTEME**
DIGITAL INTEGRATION SYSTEM FOR INTEGRATING DIAGNOSTIC IMAGING AND DATA-PROCESSING DEVICES INTO EDP SYSTEMS
SYSTEME D'INTEGRATION DIGITAL POUR INTEGRER DANS DES SYSTEMES INFORMATIQUES DES APPAREILS D'IMAGERIE ET DE TRAITEMENT DE DONNEES DIAGNOSTIQUES

(30) Priorität: 13.10.1993 DE 4334782; 05.05.1994 DE 4415818
(43) Veröffentlichungstag der Anmeldung: 31.07.1996
(73) Patentinhaber: CSB-System Software-Entwicklung & Unternehmensberatung GmbH, D-52511 Geilenkirchen (DE)
(72) Erfinder: SCHIMITZEK, Peter, D-52511 Geilenkirchen (DE)
(74) Vertreter: Haussingen, Peter
(86) Internationale Anmeldenummer: DE9401124
(87) Internationale Veröffentlichungsnummer: WO9510816

(56) Entgegenhaltungen:
- GB-A- 2 246 456
- US-A- 5 208 745
- PROCEEDINGS 5TH IEEE SYMPOSIUM ON COMPUTER-BASED MEDICAL SYSTEMS, 14. Juni 1992, DURHAM, US Seiten 156 - 169 S. FRANCHI ET AL : 'Multimedia perspectives for next generation PAC systems'
- EUROPEAN TRANSACTIONS ON TELECOMMUNICATIONS AND RELATED TECHNOLOGIES, Bd.2, Nr.1, Januar 1991, MILANO IT Seiten 5 - 19 L. BARBOSA ET AL : 'Multimedia services and applications'

## Beschreibung

Die Erfindung betrifft ein digitales Integrationssystem zur Integration von diagnostischen Bilderzeugungs- und Datenverarbeitungsgeräten in EDV-Systeme, mit welcher Bild-, Ton- und Textdatenaufnahmen und -übertragungen vorgenommen und Daten gedruckt, archiviert und analysiert werden können.

Nach der Druckschrift DE-OS 35 33 446 ist eine Lösung aufgezeigt, nach der ein medizinisches Bildarchiv geschaffen wurde, mit dem ohne weiteres und rasch Referenzbilder ebenso wie klinische Bilder betrachtet werden können, darüber hinaus besteht eine Einrichtung in der die benötigten Daten gespeichert werden und eine weitere Einrichtung ist vorhanden zur Eingabe von Wiedergewinnungsdaten entsprechend des gewünschten Referenzbildes und klinischen Bildes; eine Einrichtung zur Wiedergewinnung von mindestens eines Bildes aus den gespeicherten Bildern, sowie zum Steuern der Speichereinrichtung, der Eingabevorrichtung, der Wiedergewinnungseinrichtung und Anzeigeeinrichtung in der Weise, daß ein Referenzbild oder mehrere Referenzbilder und klinische Bilder aus den in der Speichereinrichtung gespeicherten Referenzbildern und klinischen Bildern wiedergewonnen werden und daß die wiedergewonnenen Referenzbilder und klinischen Bilder dargestellt werden.

In der Druckschrift DE-OS 35 33 466 ist ein Bildarchiv dargestellt, bei dem eine Wiedergabeweise zur Wiedergabe einer bereits aufgezeichneten Abbildung und eine erste Aufzeichnungsweise zum Aufzeichnen von Bildsignalen vorgesehen ist, während das von der Bildaufnahmevorrichtung gelieferte Bild ständig auf einer Anzeigeeinheit dargestellt wird, ist eine zweite Aufzeichnungsweise vorgesehen, bei der unmittelbar nach dem Aufzeichnen eines Bildsignales auf eine Bildplatte das gerade aufgezeichnete Bildsignal automatisch gelesen und während einer vorherbestimmten Zeitspanne auf der Anzeigeeinheit dargestellt wird. Dann wird die gerade aufgenommene Abbildung auf der Anzeigeeinheit dargestellt, um mit dem Aufzeichnungsvorgang der nächsten Abbildung zu beginnen. Das macht es möglich, leicht und schnell festzustellen, ob die aufgezeichnete Abbildung richtig aufgezeichnet wurde oder nicht.

Weiterhin ist ein Ablagesystem für Bilder aus dem medizinischen Bereich nach der Druckschrift DE-OS 35 34 065 bekannt, das aus einem Bilddatenspeicherabschnitt, einem Bilddateneingabe- und -ausgabeabschnitt zur Eingabe/Ausgabe der Bilddaten, einem Rückholabschnitt zum Speichern der Bilddaten in den Speicherabschnitt und zum Wiederholen hieraus auf der Grundlage von Rückholdaten, einem Rückholdateneingabeabschnitt zur Eingabe der Rückholdaten in den Rückholabschnitt, einem Rückholdatenabschnitt zum Speichern der Rückholdaten, wobei die Rückholdaten durch einen Informationsblock klassifiziert werden, welcher bei Einzeluntersuchung erhalten wird.

Werden Bilder aus dem medizinischen Bereich abgelegt, so werden Rückholdaten, welche für jede Untersuchung gesammelt wurden, zur Reduzierung der Menge von Rückholdaten verwendet, während bei der Reproduktion das Rückholen für jede Einzeluntersuchung ausgeführt wird, wodurch die zum Rückholen erforderliche Zeit verkürzt wird.

Alle vorgenannten Lösungen besitzen den Nachteil, daß sie nicht multimedial ausgerichtet sind. Bild-, Ton- und Textsignale sind nicht integrationsfähig. Auch eine gleichzeitige digitale Darstellung der Daten neben einer analogen Anzeige ist nicht gegeben. Diese vorgenannten Lösungssysteme haben auch den Nachteil, daß sie heterogene Systeme nicht integrieren, d. h., daß der Arzt nicht in die Lage versetzt wird, die vielfältigen Signale zu sammeln und durch die Hardware ihm nicht die Möglichkeit geboten wird, diese auf digitale Systeme zu übertragen.

Nach der Druckschrift Proceedings 5th IEEE Symposium on computer-based medical systems, 14. juni 1992, Durham; US; Seiten 156-169; S.Franchi et al.: ,,Multimedia perspectives for next generation PAC systems" ist die Lösung eines neuen Multimedia-Informations-System dargestellt, das eine Ausdehnung der früher beschriebenen Merkmale von PAC-Systemen (PAC = Patient Administration Control) impliziert. Datenbank-Strukturen werden definiert um die physikalischen Eigenschaften und den klinischen Inhalt jeder Art von multimedialen klinischen Daten zu beschreiben. Vorhandene Audio- und/oder Videosequenzen werden archiviert. Weiterhin setzt man lokal Hochgeschwindigkeitsnetze ein. Außerdem impliziert die Übertragung von Multimedia-Informationen weitere Anforderungen an das Netzwerk-Subsystem, z. B. Fähigkeiten um den Synchronverkehr zu unterstützen. Die Benutzer-Workstationen bieten Zugang und Präsentation von Multimedia-Daten. Das Hypertext-Modul wird für eine wirksame Konsultation von klinischen Daten benutzt. Diese Lösung entspricht eines Multimedia-Informationsmanagements und kann damit die Basis eines avancierten PAC-Systems schaffen.

Der Nachteil dieser Lösung besteht darin, daß sie nur einen multimedialen Zugriff auf ein bereits bestehendes Multimedialarchiv sichert bzw. nur die Möglichkeit gibt zwischen schon vorhandenen Daten mit unterschiedlichen Formaten nachträglich multimediale Beziehungen herzustellen und sie hiermit zu Datenformaten zu archivieren. Nachteilig ist, daß die multimediale Datenbank in einem LAN (Local Area Network) auf der Grundlage eines TDDI Netzwerkes realisiert ist. Eine bildgebende Telekommunikation und Telekonsultation ist nicht gegeben. Die Anforderungen an ein modernes Multimedia-Informations-System zur Sicherung hoher Transferleistungen bei einer One-Line-Übertragung von Bildsequenzen und ein größerer Datenschutz wegen der immanenten Abhörsicherheit ist nicht gegeben.

Medizinisch beurteilbare Bilder in höherer Auflösung und dem Untersuchungsablauf entsprechend kann nicht gewährleistet werden.

Ein zusätzliches optisches Erfassen von multimedialen Daten mit ihren internen Querbeziehungen ist nicht möglich. So ist ein Erfassen und eine Integration von neuen multimedialen Daten während eines Untersuchungsablaufes nicht möglich.

Auch ist es nicht gegeben, daß während des Untersuchungsablaufes die notwendigen Relationen zwischen den unterschiedlichen Daten hergestellt werden.

Um die oben genannten Nachteile des Standes der Technik zu beseitigen, ist es Aufgabe der Erfindung, eine Anordnung zur Integration von diagnostischen Bilderzeugungs- und Datenverarbeitungsgeräten in EDV-Systeme zu entwickeln, die zusätzlich zu multimedialen Daten neue Daten, auch in Form von Ton- und Bildsequenzen, erfaßt und archiviert, die extrem schnelle One-Line-Analysen direkt auf dem Bildschirm absichert sowie mit globalen Netzen korrespondiert und medizinisch beurteilbare Bilder in höherer Auflösung, schnell ausdruckbar liefert, wobei eine One-Line-Übertragung von Ton- und Bildsequenzen und Datenschutz gewährleistet wird.

Erfindungsgemäß wird die Aufgabe durch die in dem Patentanspruch angegebenen Merkmale gelöst.
Die Vorteile der Erfindung bestehen darin, daß eine direkte On-Line-Archivierung und eine On-Line-Druckmöglichkeit gegeben ist. Weiterhin wird eine intelligente Bildanalyse mit automatisiertem Werkzeugeinsatz auf verschiedenen Bildebenen auf dem A/D-Wandler mit seinem integriertem Speicher ermöglicht. Desweiteren sind multifunktionale Hardware-Schnittstellen vorhanden, um jegliche Bild-, Ton- und Textdatenübertragungseinheit anzuschließen.
Darüber hinaus ist diese Vorrichtung multimedial für alle Trägersysteme ausgerichtet und der Fachmann ist in die Lage versetzt, die vielfältigen Systeminformationen zu sammeln, wobei die Hardware die Möglichkeit besitzt, diese Signale auf digitale Systeme zu übertragen.
Mit diesen Vorteilen ist eine Standardisierung des Untersuchungsablaufes abgesichert und eine modellbasierende Auswertung der Organstrukturen im ruhenden und bewegten Zustand ist gegeben. Gleichzeitig ist eine triggergesteuerte Bildspeicherung vorhanden. Jedem berechtigten Nutzer stehen gleichzeitig alle Daten zur Verfügung. Bild-, Ton- und Textdaten sind entsprechend des Protokolls für Hochgeschwindigkeitsnetze absetzbar bzw. abforderbar. Die Archivierung beginnt schon beim Untersuchungsvorgang.

Mit dieser Vorrichtung ist die Lichtleitertechnik zur bildgebundenen Telekommunikation und Telekonsultation einsetzbar. Neben der Text-, Bild- und Tondigitalisierung ist auch die Signalaufnahme und -verarbeitung von Bewegtbildern in Farbe möglich. Eine grafikfähige Text- und Layoutverarbeitung ermöglicht die grafische Präsentation, die Reproduktion, Ausgabe und Dokumentation von Befundmaterial.
Diese Vorrichtung entspricht der modernen Herausforderung im Gesundheitswesen im Hinblick auf eine effektivere Gestaltung der Heilfürsorge aber auch für die Kostendämpfung auf diesem Gebiet und der qualitativen Kontrolle der medizinischen Leistungen.

Ein weiterer Vorteil liegt darin, daß die Flut von Informationsmasse in der heutigen Medizin, durch diese Vorrichtung zusammengetragen, miteinander verknüpft und selektiert wird und somit diese für die Therapie effektiv nutzbar ist. Das nötige EDV-Fachwissen für den medizinisch orientierten Anwender wird auf ein Minimum reduziert. Die Abläufe im Sinne eines informationstechnischen Managements bleiben im Hintergrund unbelastet für den Anwender. Die Nachvollziehbarkeit der Abläufe ist gewährleistet und es ist ein leicht durchschaubares und einfach durchführbares patientenverteiltes Informationsmanagement gegeben.

Die Druckgeschwindigkeit ist wesentlich gesteigert bei gleichzeitig stark erhöhter Bildqualitätsbereitstellung. Eine One-Line-Übertragung von Bildsequenzen und ein größerer Datenschutz wegen der immanenten Abhörsicherheit ist gegeben. Die Komprimierung und Dekomprimierung sowohl von Einzelbildern als auch von ganzen Bildsequenzen ist möglich. Videosequenzen können gezielt auf einem Videoband angesteuert werden und analoge abgespeicherte Daten sind verwaltungsfähig.

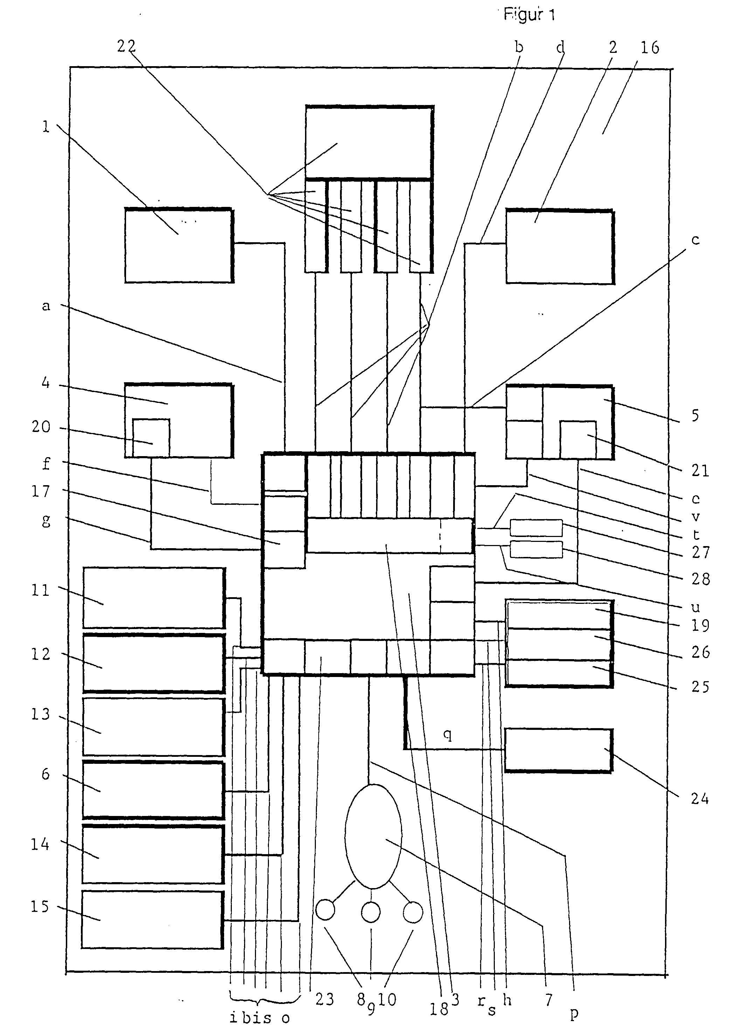

Die Erfindung wird nachstehend an Hand der Figur 1 erläutert. Das in Figur 1 dargestellte digitale Integrationssystem zur Integration von diagnostischen Bilderzeugungs- und Datenverarbeitungsgeräten in EDV-Systeme, an die jedes bestehende Untersuchungssystem angeschlossen werden kann und aus dem Menü-Monitor 1, dem Video-Monitor 2, dem Personalcomputer 3, dem Drucker 4, dem Videorecorder 5, dem -optischen Laufwerk- 6, dem Netz 7 mit PC's 8; 9; 10, dem Laufwerk A 11, den Festplatten 12; 13 und den Speichern 14; 15 besteht, ist dadurch charakterisiert, daß ein digitales Integrationssystem 16 aus dem Personalcomputer 3, der mit einem Interface 17 für den Drucker 4 im Personalcomputer 3 und mit dem Zusatzmodul für die Frame-Grabber-Karte 18 bestückt ist, aus einem zusätzlichen Fußschalter 19, aus dem Drucker 4 mit einem erweiterten Interface 20 für den Drucker 4 im Drucker 4 und aus dem Videorecorder 5 mit einem automatischen Steuersystem 21, besteht, indem der Personalcomputer 3
- mit seiner Grafik-Karte durch die Informationsleitung a mit dem Menü-Monitor 1,
- mit dem Zusatzmodul für die Frame-Grabber-Karte 18 über seine LINE IN 1 bis LINE IN 4 bzw. LINE IN n, mindestens mit einem dieser durch die Informationsleitung b mit den bestehenden bildgebenden Untersuchungssystemen 22, mindestens mit einem dieser,
- mit dem Zusatzmodul für die Frame-Grabber-Karte 18 über eine seiner LINE IN 1 bis LINE IN 4 bzw. LINE IN n mittels Informationsleitung c mit dem LINE OUT des Videorecorders 5,
- mit dem Zusatzmodul für die Frame-Grabber-Karte 18 über seine LINE OUT durch die Informationsleitung d mit dem Video-Monitor 2 bzw. Menü-Monitor 1,
- mit seinem LPT 2 über die Informationsleitung e mit dem speziellen automatischen Steuersystem 21,
- mit seinem LPT 1 über die Informationsleitung f mit dem Drucker 4,
- mit seinem erweiterten Drucker-Interface 17 für den Drucker 4 im Personalcomputer 3 durch die Informationsleitung g mit dem erweiterten Interface 20 für den Drucker 4 im Drucker 4,
- mit seiner COM 2 über die Informationsleitung h mit dem Fußschalter 19,
- über seinem SCSI mit dem optischen Laufwerk 6, dem Laufwerk A 11, der Festplatte C 12, der Festplatte D 13, dem Speicher F 14 und dem Speicher G 15 jeweils mit ihren Informationsleitungen i; k; l; m; n; o verbunden,
- mit seiner Netzkarte über die Informationsleitung p mit dem Netz 7 und dieser mit den PC's 8; 9; 10,
- mittels dem Anschluß für Hochgeschwindigkeitsnetze 23 über eine gesonderte Leitung entsprechend der Protokollvorschrift dieser Netze,
- mit dem Triggersystem 24 über die Informationsleitung p, der Tastatur 25 über die Informationsleitung r und der Maus 26 über die Informationsleitung s, sowie mit der Tonaufnahmeeinrichtung 27 und der Tonwiedergabeeinrichtung 28 über die Informationsleitung t in Wirkverbindung steht, wobei der LINE IN des Videorecorder 5 parallel zum LINE OUT des Personalcomputers 3 und dem Video-Monitor 2 bzw. Menü-Monitor 1 über die Informationsleitung v geschaltet ist.

Die Funktion ist wie folgt.
Daß, nachdem der Personalcomputer 3 eingeschaltet ist, ein betreffendes Bearbeitungssystem über die Tastatur 25 oder Maus 26 aufgerufen werden kann. Gleichzeitig wird damit ein bildgebendes Untersuchungssystem 22 oder bei Bedarf, mehrere bildgebende Untersuchungssysteme 22 am Video-Monitor 2 bzw. Menü-Monitor 1 angezeigt, wobei nach Bedarf und Erfordernis parallel dazu die Möglichkeit der Aufnahme von Tondaten (Biosignale und/oder Kommentare des Fachmannes) durch die dafür bestimmte Tonaufnahmeeinrichtung 27 bzw. über das Netz und Textdaten durch die Tastatur 25 bzw. über das Netz besteht, die gemeinsam und zeitgleich mit dem am Video-Monitor 2 bzw. Menü-Monitor 1 angezeigten bildgebendem Untersuchungssystem 22 bzw. den Untersuchungssystemen 22 im Speicher des Personalcomputers 3 zwischengespeichert werden. Anschließend wird über die Bedienung des Fußschalters 19 oder über die Bedienung der Tastatur 25 bzw. über die Bedienung der Maus 26 die Ablage der Ton-, Bild- und/oder Textdaten in eine der Positionen, Festplatte D 13, Speicher F 14, Speicher G 15 vorgenommen. Danach können bei Bedarf die Bild-, Ton- und Textdaten in der Weise zurückgeholt werden, indem der Fußschalter 19 oder die Tastatur 25 bzw. die Maus 26 bedient werden, so daß die Bild-, Ton- und Textdaten im Personalcomputer 3 anstehen, Bild und nach Bedarf der Text auf dem Video-Monitor 2 bzw. Menü-Monitor 1 erscheint und gegebenenfalls Bild und nach Bedarf Text auf dem Menü-Monitor 1 bzw. Video-Monitor 2 zu sehen sind und der Ton, wenn erforderlich, an der Tonwiedergabeeinrichtung 28 anliegt. Desweiteren können, wenn notwendig, gleichzeitig oder zeitversetzt über den Drucker 4 ein oder mehrere Bilder oder Bilder mit Text nach verschiedenen Ordnungskriterien ausgedruckt werden. Weiterhin sind mittels Videorecorder 5 Aufnahmen und Wiedergaben von hinterlegten Bild-, Text- und Tondaten durch die Bedienung der Maus 26 oder Tastatur 25 möglich.
Unter Einbeziehung eines Netzes 7 stehen die gespeicherten Bild-, Ton- und Textdaten jedem berechtigten Nutzer über die jeweiligen PC's 8; 9; 10 zur Verfügung.
Mittels eines Anschlusses für Hochgeschwindigkeitsnetze 23 im Personalcomputer 3 sind Voraussetzungen für diese Vorrichtung gegeben, diese gespeicherten Bild-, Ton- und Textdaten entsprechend des Protokolls für Hochgeschwindigkeitsnetze abzusetzen bzw. abzufordern.
Weiterhin ist dem Personalcomputer 3 ein Triggersystem 24 zugeordnet und es wird mit dieser Vorrichtung eine triggergesteuerte Bildspeicherung ermöglicht.

Desweiteren wird mit dem erweiterten Drucker-Interface 17 im Personalcomputer 3 und dem erweiterten Interface 20 im Drucker 4 eingebauten Interface die Druckgeschwindigkeit gesteigert bei gleichzeitig stark erhöhter Bildqualität, indem die Daten bereits im Personalcomputer 3 aufgearbeitet werden und der Personalcomputer-Prozessor übernimmt die Ansteuerung des Laserkopfes. Somit entfällt der bisher vorhandene langsame Datentransfer zum Drucker 4 und es wird die schnelle Prozessorleistung des Rechners genutzt. Mittels Zusatzmodul für die Frame-Grabber-Karte 18 wird die hardware-technische Zusatzkomponente zur Komprimierung und zur Dekomprimierung sowohl von Einzelbildern als auch von ganzen Bildsequenzen verwirklicht und beinhaltet auch zusätzlich hardwaretechnisch ausgeführte extrem schnelle Bildanalysewerkzeuge. Weiterhin wird mit dem automatischen Steuersystem 21 die Möglichkeit realisiert, daß der Videorecorder vom Personalcomputer 3 in der Weise angesteuert wird, daß gezielt Videosequenzen auf einem Videoband angesteuert werden und daß man auch analoge abgespeicherte Daten verwalten kann. Mit dieser Anordnung werden die speziellen Zugriffsmöglichkeiten der Frame-Grabber-Karte auf das Bildmaterial verwirklicht, um extrem schnelle One-Line-Analysen direkt auf dem Bildmaterial vorzunehmen. So z. B. können dem eigentlichen Bild mehrere Bildlayer überlagert werden.

Desweiteren ist der Einsatz der Lichtleitertechnik zur bildgebenden Telekommunikation und Telekonsultation möglich. Damit wird aufgrund der hohen Transferleistung eine One-Line-Übertragung von Bildsequenzen und ein größerer Datenschutz wegen der immanenten Abhörsicherheit erreicht.

### Verwendete Bezugszeichen

- 1: Menü-Monitor
- 2: Video-Monitor
- 3: Personalcomputer
- 4: Drucker
- 5: Videorecorder
- 6: optisches Laufwerk
- 7: Netz
- 8: PC's
- 9: PC's
- 10: PC's
- 11: Laufwerk A
- 12: Festplatte C
- 13: Festplatte D
- 14: Speicher F
- 15: Speicher G
- 16: digitales Integrationssystem
- 17: erweitertes Drucker-Interface
- 18: Zusatzmodul für die Frame-Grabber-Karte
- 19: Fußschalter
- 20: erweitertes Interface
- 21: automatisches Steuersystem
- 22: bildgebendes Untersuchungssystem
- 23: Anschluß für Hochgeschwindigkeitsnetze
- 24: Triggersystem
- 25: Tastatur
- 26: Maus
- 27: Tonaufnahmeeinrichtung
- 28: Tonwiedergabeeinrichtung
- a-v: Informationsleitung

## Patentansprüche

1. Digitales Integrationssystem (16) zur Integration von diagnostischen Bilderzeugungs- und Datenverarbeitungsgeräten in EDV-Systeme, an die bildgebende Untersuchungssysteme (22) angeschlossen werden können, bestehend aus folgenden Komponenten:
aus dem Menü-Monitor (1), aus dem Video-Monitor (2), aus dem Personalcomputer (3), aus dem Drucker (4), aus dem Videorecoder (5), aus dem Optical Disk (6), aus dem Netz (7), aus PC's (8; 9; 10), aus dem Laufwerk A (11),
aus der Festplatte C (12), aus der Festplatte D (13), aus dem Speicher F (14), aus dem Speicher G (15), aus dem Zusatzmodul für die Frame-Grabber-Karte (18), aus dem Anschluß für Hochgeschwindigkeitsnetze (23) im Personalcomputer (3), aus dem Triggersystem (24), aus der Tastatur (25), aus der Maus (26), aus der Tonaufnahmeeinrichtung (27), aus der Tonwiedergabeeinrichtung (28) und aus den Informationsleitungen (a bis v),
wobei der Personalcomputer (3) mit einem Standardinterface (LPT1) über eine Informationsleitung (f) mit dem Drucker (4) verbunden ist, mit seiner SCSI Schnittstelle über die jeweiligen Informationsleitungen (i-o) mit der Optical Disc (6), dem Laufwerk A (11), der Festplatte C (12), der Festplatte D (13), dem Speicher F (14) und dem Speicher G (15) verbunden ist, mit seiner Netzkarte über eine Informationsleitung (p) mit einem Netz (7) und an diesem Netz angeschlossenen weiteren PC's (8; 9; 10) verbunden ist,
mit einem Anschluß für Hochgeschwindigkeitsnetze (23), über eine gesonderte Leitung entsprechend der Protokollvorschrift dieser Netze mit Hochgeschwindigkeitsnetzen verbunden ist, mit Anschlüssen über die jeweilige Informationsleitungen (r; s) mit einer Tastatur (25) und einer Maus (26) verbunden ist,
dadurch gekennzeichnet, daß
der Personalcomputer (3) mit einem erweiterten Drucker-Interface (17) bestückt ist, das über eine Informationsleitung (g) direkt mit einem erweiterten Interface (20) im Drucker (4) verbunden ist,
wobei gleichzeitig oder zeitversetzt über den Drucker (4) ein oder mehrere Bilder oder Bilder mit Text nach verschiedenen Ordnungskriterien ausdruckbar sind,
mit einem Standard-Interface (LPT2) über eine Informationsleitung (e) mit einem automatischen Steuerungssystem (21) des Videorecorders (5) verbunden ist, mit einem weiteren Standard-Interface (COM2) über eine Informationsleitung (h) mit einem Fußschalter (19) verbunden ist, über eine Informationsleitung (q) mit einem Triggersystem (24) verbunden ist, das eine triggergesteuerte Bildspeicherung ermöglicht,
das Zusatzmodul für die Frame-Gabber-Karte (18) mit mindestens einem seiner Eingänge (LINE IN 1 - LINE IN 4) über mindestens eine Informationsleitung (b) mit mindestens einem bildgebenden Untersuchungssystem (22) verbunden ist,
mit mindestens einem seiner Eingänge (LINE IN 1 -LINE IN 4) über eine Informationsleitung (c) mit dem LINE OUT des Videorecorders (5) verbunden ist,
mit mindestens einem seiner Eingänge (LINE IN 1 - LINE IN 4) über eine Informationsleitung (d) mit dem Video-Monitor (2) verbunden ist,
über eine Informationsleitung (t) mit einer Tonaufnahmeeinrichtung (27) in Wirkverbindung steht,
über eine Informationsleitung (u) mit einer Tonwiedergabeeinrichtung (28) in Wirkverbindung steht,
und daß die LINE IN des Videorecorders (5) parallel zum LINE OUT des Personalcomputers (3) und dem Video-Monitor (2) über die Informationsleitung (v) geschaltet ist;
wobei folgende Funktionen vom digitalen Integrationssystem (16) ausgeführt werden:
nach Einschalten des Personalcomputers (3) wird ein Bearbeitungssystem über die Eingabeelemente (19; 25; 26) aufgerufen und gleichzeitig mindestens ein bildgebendes Untersuchungssystem (22) auf dem Video-Monitor (2) angezeigt, wobei gemeinsam und zeitgleich eingehende multimediale Daten (Bild-/Ton-/Textdaten) im Speicher des Personalcomputers (3) zwischengespeichert werden,
danach können über die Bedienung der Eingabeelemente (19; 25; 26) die multimedialen Daten (Bild-/Ton-/Textdaten) in das Langzeitarchiv (5; 6; 12-15) übernommen werden und wiederabgerufen werden und stehen über das Netz (7) jedem berechtigten Nutzer über PC's (8; 9; 10) zur Verfügung.

## Claims

1. Digital integration system (16) for the integration of diagnostic image-generation and data-processing units within EDP systems, to which image-providing examination systems can be connected and which consists of the following components:
menu monitor (1), video monitor (2), personal computer (3), printer (4), video recorder (5), optical disk (6), the network (7) with PCs (8; 9; 10), drive A (11), hard disk C (12),
hard disk D (13), memory F (14), memory G (15), add-on module for frame grabber card (18), port for high-speed networks (23) in the personal computer (3), trigger system (24), keyboard (25), mouse (26), sound recording facility (27), sound reproduction facility (28) and information lines (a to v),
where the personal computer (3) is connected by a standard interface (LPT1) via an information line (f) to the printer (4), by its SCSI interface via the appropriate information lines (i to o) to the optical disk (6), drive A (11), hard disk C (12), hard disk D (13), memory F (14) and memory G (15), by its network card via an information line (p) to the network (7) and to the PCs attached to this network (8; 9; 10);
it is also connected to high-speed networks using its port for high speed networks (23) via a separate line in accordance with the protocol allocation rules for this network, and is connected by ports via the respective information lines (r; s) to a keyboard (25) and a mouse (26),
characterised by the fact that
the personal computer (3) is equipped with an extended printer interface (17) which is directly connected to an extended interface (20) in the printer (4) via an information line (g),
where one or more images or images with text can be printed out on the printer (4) simultaneously or in sequence, according to different classification criteria,
is connected by a standard interface (LPT2) via an information line (e) to an automatic control system (21) of the video recorder (5), by a further standard interface (COM 2) via an information line (h) to a foot switch (19), and via an information line (q) to a trigger system (24) which enables trigger-controlled image storage
the add-on module for the frame grabber card (18) is connected by at least one of its inputs (LINE IN 1 - LINE IN 4) via at least one information line (b) to at least one image-providing examination system (22),
it is connected by at least one of its inputs (LINE IN 1 - LINE IN 4) via an information line (c) to the LINE OUT of the video recorder (5),
is connected by at least one of its inputs (LINE IN 1 - LINE IN 4) via an information line (d) to the video monitor (2),
it is in operative connection via an information line (t) with a sound recording facility (27),
it is in operative connection via an information line (u) with a sound reproduction facility (28),
and that the LINE IN of the video recorder (5) is switched parallel to the LINE OUT of the personal computer (3) and the video monitor (2) via information line (v),
whereby the following functions of the digital integration system (16) are executed:
when the personal computer (3) is switched on, the processing system is called up via the input elements (19; 25; 26) and at least one image-providing examination system (22) is simultaneously displayed on the video monitor (2),
where jointly and simultaneously incoming multimedia data (image, sound, text data) is buffered in the memory of the personal computer (3).
The input elements (19; 25; 26) can then be used to transfer the multimedia data (image, sound, text data) to the long-term archive (5; 6; 12-15) and to call it up again, and it will be available on the network (7) to every authorised user via the PCs (8; 9; 10).

## Revendications

1. Système d'intégration numérique (16) destiné à intégrer dans des systèmes informatiques des appareils de diagnostics de traitement de données et de production d'images auxquels des systèmes d'analyse fournissant des images (22) peuvent être connectés, se composant comme suit :
du moniteur menu (1), du moniteur vidéo (2), du PC (3), de l'imprimante (4), du magnétoscope (5) du disque optique numérique (6) , du réseau (7), de plusieurs PC (8;9;10), du lecteur A (11), du disque dur C (12), du disque dur D (13), de la mémoire F (14), de la mémoire G (15), du module additionnel pour la carte d'acquisition de trame (18), de la connexion pour des réseaux à grande vitesse (23) dans le PC (3), du système de déclenchement (24), du clavier (25), de la souris (26), du dispositif de prise de son (27), du dispositif de reproduction sonore (28) et des lignes de transmission d'informations (a à v),
le PC (3) étant relié avec une interface standard (LPT1) à une imprimante (4) par l'intermédiaire d'une ligne de transmission d'informations (f), étant relié avec son interface SCSI par l'intermédiaire des lignes de transmission d'informations respectives (i-o) au disque optique numérique (6), au lecteur A (11), au disque dur C (12), au disque dur D (13), à la mémoire F (14) et la mémoire G (15), et étant relié avec sa carte réseau par l'intermédiaire d'une ligne de transmission d'informations au réseau (7) et à d'autres PC (8;9;10) connectés à ce réseau,
étant relié avec une connexion pour réseaux à grande vitesse (23) par l'intermédiaire d'une ligne de transmission séparée à des réseaux à grande vitesse conformément au règlement de protocole de ces réseaux, étant relié avec des connexions par l'intermédiaire des lignes de transmission d'informations respectives (r;s) à un clavier (25) et à une souris (26),
**caractérisé par le fait que**
le PC (3) est équipé d'une interface d'imprimante élargie (17) qui est reliée dans l'imprimante (4) directement à une interface élargie (20) par l'intermédiaire d'une ligne de transmission d'informations (g),
une ou plusieurs images ou une ou plusieurs images comportant du texte étant susceptibles d'être imprimées, en même temps ou avec décalage dans le temps, par l'intermédiaire de l'imprimante (4) selon des critères d'ordre différents,
le PC (3) est relié avec une interface standard (LPT2) par l'intermédiaire d'une ligne de transmission d'informations (e) à un système de commande automatique (21) du magnétoscope (5), étant relié avec une autre interface standard (COM2) par l'intermédiaire d'une ligne de transmission d'informations (h) à un commutateur à pédale (19), étant relié par l'intermédiaire d'une ligne de transmission d'informations (q) à un système de déclenchement (24) permettant une mémorisation des images commandée par déclencheur,
le module additionnel pour la carte d'acquisition de trame (18) est relié avec au moins l'une de ses entrées (LINE IN 1 - LINE IN 4) par l'intermédiaire d'au moins une ligne de transmission d'informations (b) à au moins un système d'analyse fournissant des images (22),
étant relié avec au moins l'une de ses entrées (LINE IN 1 - LINE IN 4) par l'intermédiaire d'une ligne de transmission d'informations (c) à la ligne LINE OUT du magnétoscope (5),
étant relié avec au moins l'une de ses entrées (LINE IN 1 - LINE IN 4) par l'intermédiaire d'une ligne de transmission d'informations (d) au moniteur vidéo (2),
étant relié activement par l'intermédiaire d'une ligne de transmission d'informations (t) à un dispositif de prise de son (27),
étant relié activement par l'intermédiaire d'une ligne de transmission d'informations (u) à un dispositif de reproduction sonore (28),
et que la ligne LINE IN du magnétoscope (5) est appliquée en parallèle par rapport à la ligne LINE OUT du PC (3) et du moniteur vidéo (2) par l'intermédiaire de la ligne de transmission d'informations (v) ;
les fonctions suivantes étant exécutées par le système d'intégration numérique (16) :
après l'allumage PC (3), un système de traitement est appelé par l'intermédiaire des éléments d'entrée (19;25;26), et au moins un système d'analyse fournissant des images (22) est indiqué en même temps sur le moniteur vidéo (2), des données multimédia (données sur l'image, le son et le texte) étant mémorisées temporairement dans la mémoire du PC (3),
après cela, les données multimédia (données sur l'image, le son et le texte) peuvent être mises dans l'archivage longue durée (5;6;12-15) par l'intermédiaire de l'actionnement des éléments d'entrée (19;25;26) et être appelées à nouveau et elles sont alors disponibles par le réseau pour chaque utilisateur autorisé par l'intermédiaire des PC (8;9;10).
